# EUROPEAN PATENT APPLICATION

(11) **EP 3 649 924 A1**
(43) Date of publication of application: **13.05.2020**
(21) Application number: 18205681.2
(22) Date of filing: 12.11.2018
(51) Int. Cl.: A61B 5/00

(54) **PAIN REDUCTION DURING PATIENT TRANSFER**

(71) Applicant: Koninklijke Philips N.V., 5656 AG Eindhoven (NL)
(72) Inventor: WEISS, Steffen, 5656 AE Eindhoven (NL); VUPPALA, Sunil Kumar, 5656 AE Eindhoven (NL); JOHNSON, Mark Thomas, 5656 AE Eindhoven (NL); HELLE, Michael Günter, 5656 AE Eindhoven (NL)
(74) Representative: de Haan, Poul Erik

(57) **Abstract**

In the current diagnostic imaging workflow, transfer of handicapped patients between a hospital bed to a patient support of an imaging system and back is performed manually, which may be physically exhausting for staff and uncomfortable for the patient being transferred. Furthermore, this should be avoided in an autonomous scanning environment. Accordingly, the present application proposes an approach for enabling patient pain detection and reduction when using a patient transfer device configured to transfer a patient between a first and a second patient support.

## Description

### FIELD OF THE INVENTION

This application relates generally to a device for enabling patient pain detection and reduction when using a patient transfer device configured to transfer a patient between a first and a second patient support, and an associated system, method, computer program element, and computer readable medium.

### BACKGROUND OF THE INVENTION

In the current diagnostic imaging workflow, the transfer of handicapped patients from the hospital bed to the patient support of the imaging system (and back) is typically performed manually. This is physically tiring for staff, and sometimes uncomfortable for a patient. The present trend is towards imaging suites requiring fewer human operatives, reducing the number of staff available for patient repositioning.

US 2016/0255966 A1 discusses a system for adjusting a body support including a body support having an adjustable layer and a plurality of sensors, and a processing system in communication with the plurality of sensors and the adjustable layer. However, such a system can be further improved.

### SUMMARY OF THE INVENTION

Accordingly, it would be advantageous to provide an improved technique for reducing or removing pain experienced by patient during the transfer of the patient between patient supports.

According to a first aspect, there is provided a device for enabling patient pain detection and reduction when using a patient transfer device configured to transfer a patient between a first and a second patient support comprising an input unit, a processing unit, and an output unit.

The input unit is configured to obtain, via one or more sensors, a first signal representing a proxy for a pain level experienced by a patient before, during, or after the transfer of the patient using a patient transfer device between the first and the second patient support.

The processing unit is configured to monitor the first signal representing a proxy for pain level, to detect a change in the first signal from a first level, to a second level indicating that the patient is experiencing a greater degree of pain.

The output unit is configured to transmit an adaptation signal to a patient transfer device to cause the patient transfer device to perform a first physical adaptation, to change the rate of the patient transfer, or to halt the patient transfer in response to the change in the first signal to a second level indicating that the patient is experiencing a greater degree of pain.

Accordingly, a patient may be monitored for subtle (or more obvious) signs of discomfort before, during, and/or after a transfer between a first and second patient support so that the transfer can be adapted or stopped based on the detected signs of discomfort. Also in a standard non-autonomous imaging suite some patients might be reticent about indicating that they are experiencing pain during a transfer, and this needless pain can be prevented by monitoring for subtle signs of discomfort.

Optionally, the first signal received by the input unit comprises a vital sign of the patient comprised of: galvanic skin conductance, heart rate, or breathing rate.

Accordingly, subtle signs of discomfort that are not immediately visible to medical personnel involved with the patient transfer can be detected.

Optionally, the input unit is further configured to receive a video signal from a video camera or a depth camera monitoring the patient transfer area. The first signal is derived from at least a portion of the video signal and represent at least a posture of the patient, a facial expression of the patient, an eye movement, and/or a change in pupil size of the patient.

Accordingly, signs of discomfort that can also be easily monitored by medical personnel may be automatically used to influence the determination of whether or not the patient is experiencing pain. This may enable a patient transfer to be performed with fewer staff, for example.

Optionally, the processing unit is further configured, when detecting, via the processor, a change in the first signal from the first level, to the second level, to compare the first signal to a first example signal representing an absence of pain, and to compare the first signal to a second example signal representing the presence of pain.

Accordingly, an accurate library or database of signals representing the onset of pain can be provided for, for example, transfers using different types of patient transfer device, different types of injury or medical condition, different transfer orientations, and the like. The accuracy of the determination of the onset of patient pain can be further improved.

Optionally, the input unit is further configured to obtain a patient data record of the patient prior to the transfer of the patient. The processing unit is further configured to detect the change in the first signal between the first and the second levels based additionally on a portion of the data comprised in the patient data record.

Accordingly, the determination of the onset of patient pain during a patient transfer can be customised to an individual patient stored on a previous occasion.

Optionally, the output unit is further configured to transmit the adaptation signal to perform a first physical adaptation of a patient transfer device wherein the adaptation signal causes a patient transfer unit to initiate an actuator configured to transfer the patient between the first patient support and the second patient support.

Accordingly, the position (configuration) of a patient transfer device can be adjusted to ensure that a patient experiences a reduced, or zero degree of pain before, and/or during, and/or after a patient transfer.

Optionally, the input unit is further configured to receive a second signal representing a location of the first physical adaptation of the patient transfer device relative to the patient, and to receive a third signal representing a proxy for a pain level experienced by a patient after a second physical adaptation of the patient transfer device and a fourth signal representing a location of the second physical adaptation of the patient transfer device relative to the patient.

The processing unit is further configured to associate the second signal and the first signal, to perform the second physical adaptation of the patient transfer device, to associate the third signal and the fourth signal.

The output unit is further configured to transmit a fifth signal to perform a third physical adaptation of the patient transfer device based on the change of the first and third signals.

Accordingly, the presence of pain may be localised based on feedback from the patient transfer device. In other words, the setting of a patient transfer device into various different positions can be compared to an increase or decrease in patient pain monitored by the input device. Settings leading to a minimisation of patient pain can be calculated and transmitted to the patient transfer device. For example, in a patient support comprised of a plurality of automatically inflatable honeycombs, an index matrix representing the state of deflation or inflation of the honeycombs could be provided as the localisation signal, for example. This localisation signal may be benchmarked to the level of pain at that localisation signal. Then, a further incremental adaptation of the patient support is made (transition to a third signal representing, for example, a change in the inflation or deflation state of the inflatable honeycombs) and a change in pain level may be monitored. Based on this change, the localisation setting of the support can be put into an improved or optimum setting of the support.

Optionally, the output unit is configured to transmit the fifth signal to the patient transfer device to perform a physical adaptation of the patient transfer device comprising one or more of: transmitting a signal to (i) halt or reverse an inclination of a laterally tilting patient transfer device, to (ii) halt or reverse the adjustment of a plurality of lamellae within a patient transfer device, to (iii) decrease or increase the resilience of a subset of patient support actuator cells; or to halt or reverse a plurality of rollers within a patient transfer device.

Accordingly, the technique discussed herein may be applied to a wide variety of types of patient transfer device.

According to a second aspect, there is provided a system for patient pain detection and reduction during a transfer between a first and a second patient support using a patient transfer device. The system comprises:
- a patient transfer device configured to move a patient in-between a first and a second patient support;
- a one or more sensors configured to obtain a first signal representing a proxy for a pain level experienced by a patient associated a patient transfer in-between the first and a second patient supports, and/or when the patient is stationary on the first or second patient supports; and
- a device as defined in the first aspect one of its embodiments configured to output an adaptation signal to the patient transfer device to generate a physical adaptation of the patient transfer device, to change the rate of the patient transfer, or to halt the patient transfer device, in response to the change in the signal to a second level indicating that the patient has begun to experience pain.

Optionally, in the system of the second aspect the one or more sensors comprise one or more of a galvanic skin conductance monitor, a heart rate monitor, a breathing rate monitor.

Optionally, in the system of the second aspect, the one or more sensors comprise a video camera and/or a depth camera, wherein the first signal is derived from a portion of a video signal representing at least a posture of the patient, a facial expression of the patient, or an eye movement or change in pupil size of the patient.

Optionally, in the system the second aspect, the patient transfer device comprises a resilient member having a plurality of longitudinal gas-tight pockets capable of sequenced inflation to generate a surface wave for transferring the patient between the first and the second patient support, wherein performing a physical adaptation of the patient transfer device further comprises:
inflating a first subset of the longitudinal gas-tight pockets of the plurality of longitudinal gas-tight pockets; and
if the processor does not detect a change in the signal from the first level to a second level, deflating the first subset of the longitudinal gas-tight pockets of the plurality of longitudinal gas-tight pockets and inflating a second subset of the longitudinal gas-tight pockets of the plurality of longitudinal gas-tight pockets to transfer the patient a unit of distance between the first and second patient supports.

Accordingly, the system may employ a patient transfer device that smoothly transfers the patient using a surface wave generated by the inflation and deflation of, for example, tubular-shaped resilient and gas-tight silicone cavities.

According to a third aspect, there is provided a method for patient pain detection and reduction before, during, or after a transfer between a first and a second patient support using a patient transfer device. The method comprises:
a) obtaining, via one or more sensors, a first signal representing a proxy for a pain level experienced by a patient before, during, or after the transfer of the patient using a patient transfer device between the first and the second patient support ;
b) monitoring, via a processor, the first signal representing a proxy for pain level ;
c) detecting, via the processor, a change in the first signal from a first level, to a second level indicating that the patient is experiencing a greater degree of pain
d) performing, via an output device, a first physical adaptation of the patient transfer device, changing the rate of the patient transfer, or halting the patient transfer device, in response to the change in the first signal to a second level indicating that the patient is experiencing a greater degree of pain.

According to a fourth aspect, there is provided a computer program element comprising instructions which, when executed by a computer processor, causes the computer processor to perform the steps of claim.

According to fifth aspect, there is provided a computer readable medium comprising the computer program element as defined in claim.

In this application, the term "proxy for a pain level experienced by a patient" refers to a biometric signal provided by the patient indicative of the onset of pain before, during, or after physically moving the patient relative to a patient support. Such a proxy may comprise one signal, or may comprise an ensemble of signals. Such signals are, for example, a vital sign, such as heart rate, breathing rate, and and/or galvanic skin conductance of the patient, for example. Furthermore, such a proxy may comprise visual indications such as a measured facial expression and/or the detection of cramping and/or a spasm of the patient using video equipment. Substantially any biometric indication that can be detected during a patient transfer and that can be compared to a clinically accepted pain scale suitable for use as such a proxy.

In this application, the term "degree of pain" refers to a continuous or discrete metric defining the increase or decrease in pain experienced by a patient during a transfer from zero pain to intolerable pain. Of course, at the beginning of the transfer the patient may already be experiencing a slight degree of pain caused by existing injuries. Likewise, a patient may not experience intolerable pain during transfer but only an increase in discomfort. However, monitoring a change in the degree of pain can provide an indication that the position of a patient during the transfer needs to be adjusted, or that the patient transfer and needs to be halted. Optionally, the "degree of pain" may be indexed to, or based on, an existing clinical indicator such as the "Wong-Baker" FACES pain rating scale (in the case of video detection of a patient's face during the patient transfer), for example.

In this application, the term "patient transfer device" generically refers to a means for moving a patient between a first and a second patient support in an automatic or assisted way (automatic transfer may be completed using only the patient transfer device, where is assisted transfer additionally implies the presence of a medical professional to direct or hold a patient during the transfer). For example, during a MRI scan, the first patient support is a trolley or a hospital bed having an automatic or assisted transfer function, and the second patient support is the patient bed of the MRI scanner. As such, and as will be described in more detail herein, a patient transfer device may comprise a trolley that is laterally tiltable to enable the patient to be rolled onto another surface. The patient transfer device may comprise a semi-rigid surface with inflatable actuators, optionally in a linear or honeycomb pattern, and/or tiltable lamellae, that can be placed on top of a first and second patient support. Adjusting the inflatable actuators in a particular sequence enables or assists transfer of the patient between a first and second patient support. The patient transfer device may comprise arrays of rollers, or wheels. Furthermore, the patient transfer device may comprise a miniature robotic crane attachable to a sling that lifts the patient. A commercial example of a patient transfer device is the "PowerNurse (TM)" by Astir Technologies (TM), although a skilled person will know of many other types. In all of these cases, it will be appreciated that a patient transfer device can receive at least an input signal to begin or to stop the movement (control the transfer) of the patient inbetween a first and a second patient support.

In this application, the term "physical adaptation" referring to a patient transfer device is dependent on the type of patient transfer device referenced. For example, a patient transfer device having an array inflatable honeycomb actuators for supporting a patient undergoes a "physical adaptation" if one or more inflatable honeycombs are deflated or inflated. A patient transfer device comprising a tiltable lateral bed undergoes a physical adaptation if the angle of the bed is tilted with respect to the horizontal.

Accordingly, a gist of the invention is that a system for automatic transfer of a patient is proposed enabling the automatic detection and the automatic relief of patient pain during the transfer of a patient between a first patient support and a second patient support, based on the input from one or a plurality of sensors. The technique may be applied when transferring a patient from a trolley into a diagnostic imaging system such as an MRI, CT, or PET scanner, for example, however the application is not so limited and may also apply to the transfer of a patient between a first and a second bed, for example. The technique is applicable to a wide range of different patient transfer devices that are capable of automatic control (in other words, have a control interface).

### BRIEF DESCRIPTION OF THE DRAWINGS

Exemplary embodiments will be described with reference to the following drawings:
Figure 1 schematically illustrates a device in accordance with the first aspect.
Figures 2a) and b) schematically illustrate a side-view of an embodiment of a tiltable patient transfer device.
Figures 3a)-c) schematically illustrates side-views of an embodiment of a patient transfer device with a lamellar structure in different configurations.
Figure 3d) schematically illustrates a plan view of a patient transfer device comprised of a plurality of inflatable cells.
Figures 4a) to 4e) schematically illustrate a side view of an embodiment of a patient transfer device having sequentially inflatable longitudinal pockets.
Figure 5 schematically illustrates a system according to the second aspect.
Figure 6 schematically illustrates a method according to the third aspect.

### DETAILED DESCRIPTION OF EMBODIMENTS

A workflow for diagnostic imaging examinations using CT, MRI, or PET (and hybrid systems) includes the transfer of handicapped patients from the hospital bed (first patient support) to the patient support of the imaging system (second patient support) before the examination, and back into the bed after the examination. In standard clinical practice, several staff members lift and pull a patient manually from the first patient support to the second patient support on a bed sheet. In rare cases, helper devices may be used (for example manual rolling slider aids or motorised rolling aid such as the PowerNurse (TM) by Astir Technologies.

However, the manual transfer of patient using, for example, a bed sheet requires several staff members to the present and to work together. The process can be physically exhausting and not all staff members may be suited to assist. Furthermore, prior-art transfer methods maybe to a certain degree painful, or at least uncomfortable for a patient (especially when the patient is already suffering from an injury). Furthermore, a trend in imaging systems is to enable such systems to operate with fewer human operators (more autonomously). In a completely autonomous scanning environment, there is a need for automatic patient transfer. However, whether the transfer method is partially assisted or fully autonomous, it is important to transfer the patient in a careful and sensitive way and to minimise their exposure to pain.

In a situation when patients are moved in an autonomous scanning environment (or partially assisted scanning environment) there may be a situation where a patient cannot easily express their level of pain, and the areas in which pain is most severe, to a nearby professional.

According to a first aspect, there is provided a device 10 for enabling patient pain detection and reduction when using a patient transfer device configured to transfer a patient between a first and a second patient support. The device comprises:
- an input unit 12;
- a processing unit 14; and
- an output unit 16.

The input unit 12 is configured to obtain, via one or more sensors, a first signal representing a proxy for a pain level experienced by a patient before, during, or after the transfer of the patient using a patient transfer device between the first and the second patient support;

The processing unit 14 is configured to monitor the first signal representing a proxy for pain level, to detect a change in the first signal from a first level, to a second level indicating that the patient is experiencing a greater degree of pain; and

The output unit 16 is configured to transmit an adaptation signal to a patient transfer device to cause the patient transfer device to perform a first physical adaptation, to change the rate of the patient transfer, or to halt the patient transfer in response to the change in the first signal to a second level indicating that the patient is experiencing a greater degree of pain.

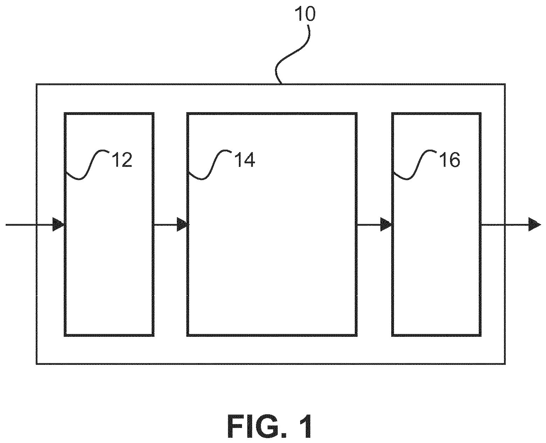

Figure 1 schematically illustrates a device 10 according to the first aspect. The device 10 may be implemented as an embedded computing device or on a personal computer, for example.

The input unit 12 is configured to receive data from range of sensors. The data may be a video feed having a field of view that covers the transfer area between a first and a second patient support by a patient transfer device, for example. The data of may be one of a number of physiological signals such as heart rate, galvanic conductance the skin, and/or breathing rate representing a proxy the pain level experienced by a patient. As such, the input unit 12 is, in an example, implemented as an Ethernet interface, a USB (TM) interface, a wireless interface such as a WiFi (TM) or Bluetooth (TM) or any comparable data transfer interface enabling data transfer between input peripherals and the processing unit 14.

The processing unit 14 is capable of processing data obtained from the input unit 12 to obtain a conclusion about the amount of pain experienced by patient during a patient transfer between a first patient support and a second patient support, and to generate output commands for transmitting to an output unit 16 accordingly. Thus, the processing unit 14 may comprise a general-purpose processing unit, a graphics processing unit (GPU), a microcontroller and/or microprocessor, a field programmable gate array (FPGA), a digital signal processor (DSP), and equivalent circuitry, alone or in combination. Furthermore, such processing unit(s) 14 may be connected to volatile or non-volatile storage, display interfaces, communication interfaces and the like as known to a person skilled in the art. Of course, a relatively low-intensity computing task such as monitoring heart rate and comparing it to a lookup table of standard values to obtain a proxy for pain level experienced by a patient might only require a microprocessor. A relatively high-intensity computing task, such as monitoring a live video feed of a patient transfer area in real-time for signs and performing image processing algorithms on the live video feed to derive signals representing a proxy for pain level might require a powerful general-purpose processing unit, in combination with a graphics processing unit and/or a field programmable gate array. A skilled person will appreciate that the implantation of the processing unit 14 is dependent on the compute intensity and latency requirements implied by the selection of signals used to represent a proxy for pain level in a particular implementation.

The output unit 16 communicates to an external patient transfer device in order to control it to provide a reduction in the amount of pain that a patient is experiencing. In a simple example, if the patient transfer device is a laterally tiltable mattress (to enable support staff to roll or to slide a patient more easily onto second patient support) then a signal communicated by the output unit 16 would be an inclination instruction transmitted to a motor or actuator of the laterally tiltable mattress. In this simple example, if the processing unit 14 detected that a patient's heart rate was rising at an unacceptable rate with the tilting of the bed (indicating onset of pain), the processing unit 14 would send a command via the output unit 16 to stop inclining the laterally tiltable mattress, and/or to reduce inclination of laterally tiltable mattress to a position where the patient was more comfortable (had a lower heart rate).

Accordingly, a pain sensor may be used to generate a physical adaptation of a patient transfer device until pain is sufficiently relieved. The device 10 for enabling patient pain detection and reduction may, in the embodiment, the able to measure acute pain of a type encountered by physical movement of an injured patient.

Optionally, the pain sensor is a galvanic skin conductance detector. Galvanic skin conductance is a vital sign that rapidly response to acute pain (on the order of milliseconds). Such a detector comprises at least two electrodes and a high impedance amplifier attached to, for example, a part of the patient's body (fingers, the wrist, or another suitable body part). A galvanic skin conductance detector has the advantage that a rapid and large increase of skin conductance is captured by the device 10 on a timescale of milliseconds to around one second after an acute pain event. This enables corrective action (physically adapting the patient transfer device) to be taken before the patient suffers prolonged pain.

Optionally, the galvanic skin conductance detector is integrated into either the first and/or the second patient support (for example, a portion of the side-rail of the first patient support may comprise at least two exposed bare-metal electrodes). Optionally, the galvanic skin conductance detector may be integrated into an elasticated bracelet worn by the patient on the wrist or ankle, for example.

Optionally, the proxy for pain level may be derived wholly or partially using the heart rate of the patient. A cardiogram may be obtained from the data output of an existing heart-rate monitor, for example.

Alternatively, the heart rate may be detected using a vital signs camera that can detect micro-blushes in the face of the patient, or calculate the heart rate or breathing rate using image processing.

Optionally, the proxy for pain level may be derived wholly or partially using the breathing rate of the patient. Accordingly, the patient may wear a breathing monitoring belt or a spirometer mask during the transfer between the first and second patient supports, for example.

Accordingly, the processing unit 14 is configured to implement digital signal processing algorithms capable of detecting an acute or more gradual build-up of pain using one or more of the galvanic skin conductance, heartrate, or breathing rate. For example, the processing unit 14 may, when detecting a change in the first signal from the first level, to the second level, to compare the first signal to a first example signal representing an absence of pain, and to compare the first signal to a second example signal representing the presence of pain.

For example, the processing unit 14 may store or have access to a library of first example signals representing galvanic skin conductance in situation where no pain is present, and second example signals representing the galvanic skin conductance at the onset of acute pain. The processing unit 14 may apply a real-time or near-time correlation algorithm over a relatively short time window (for example, 50 milliseconds) to the first signal representing a proxy the pain level and a second example signal representing galvanic skin conductance of the onset of acute pain. When the correlation between these two signals increases to a preset level, the processing unit 14 has discovered a second level indicating that the patient is experiencing greater degree of pain. Of course, the correlation function is only one way of comparing the first signal with the first and/or second example signals, and a skilled person will be able to apply many more.

Optionally, the comparison of the first signal representing a proxy for pain level with the first and/or second example signals is performed by a trained model that has been obtained using a machine learning (ML) technique. For example, a library of galvanic skin conductance traces, and/or heart rates, and/or breathing rates in different clinically labelled pain conditions can be used to provide a trained model to compare the first signal representing a proxy for pain level to indicate that the first signal has reached the second level indicating that the patient is experiencing greater degree of pain.

Optionally, at least one video camera and/or at least one depth camera having a field of view containing the area in which the patient transfer between the first patient support and second patient support occurs may be provided. The video camera is configured to provide a video signal to the input unit 12. The processing unit 14 is configured to pre-process the video signal so that information relevant for determining a proxy pain signal can be extracted from the pre-processed video signal. For example, the pre-processing stage may comprise colour and/or intensity or contrast adjustment. The pre-processing stage may comprise a feature extraction stage.

Optionally, the processing unit 14 is configured, using image processing algorithms, to record an initial posture of a patient (before patient transfer) and to track changes in the patient's posture relative to the initial posture during the patient transfer from the first patient support to the second patient support. Optionally, the processing unit 14 is configured, using image processing algorithms, to analyse a patient's facial expression for rapid changes in facial indicators of pain, for example changes in head orientation, wrinkle distribution, pupil size, mouth shape, and the like. Optionally, the processing unit 40 is configured, using an image processing algorithm, to extract the orientation of the patient arms, legs, and/or spine and to detect sudden changes in the relative and relation of these body parts indicating an acute or gradual onset of pain. The output of such image processing algorithms can be provided as a first signal representing a proxy for the pain level. The depth camera may be used in an analogous way.

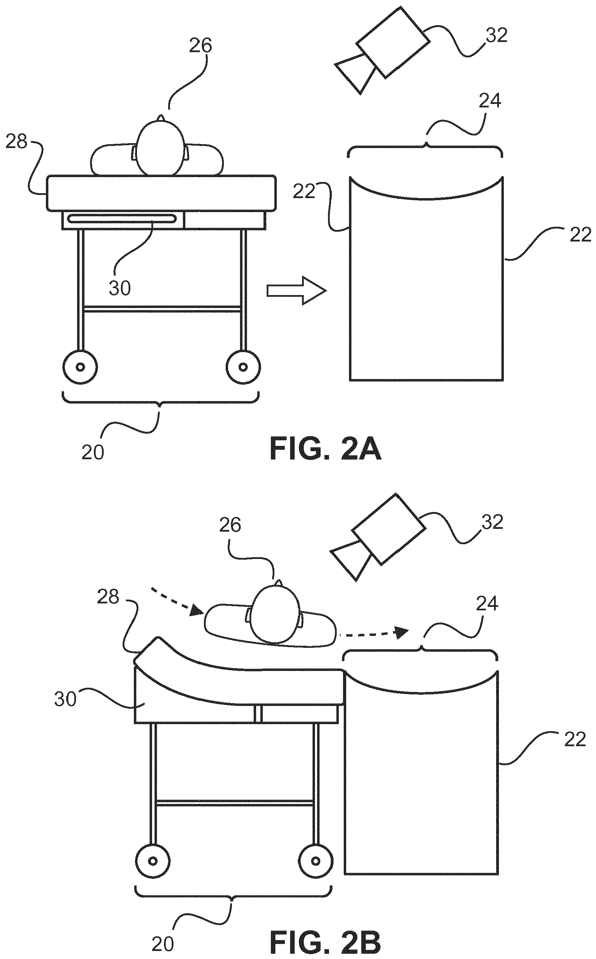

Figures 2a) and b) schematically illustrate a side-view of an embodiment of a tiltable patient transfer device.

Figure 2a) schematically illustrates a first patient support (such as a portable and automatically tiltable patient bed) 20 and a second patient support 22 (such as a CT-scanner support). In the illustrated embodiment, the CT scan support comprises a concave patient support region 24, but it may also be flat or cushioned. The patient 26 is supported on a flexible mattress 28. Underneath one side of flexible mattress is a deflated cushion 30. The patient transfer area is monitored using a video camera 32 having a field of view that covers the entire patient transfer area, or alternatively can track the patient as they are moved between the first and second patient supports.

Figure 2b) schematically illustrates the first patient support 20 after it has been moved into contact with the second patient support 22 during a patient transfer from the first patient support 22 the second patient support 22. In this example, the inflatable cushion 30 is in an inflated state and has a wedge shape. This causes one side of the flexible mattress 28 to be tilted upwards to enable the patient 26 to be rolled along path 34 onto the surface of the second patient support 24. In this example, the video camera 32 detects visual signs representing a proxy for pain level experienced by patient during transfer. If the first signal representing a proxy for pain level changes to exceed a second level, indicating that the patient is experiencing too much pain during the transfer patient, the processing unit 14 issues a command to halt the inflation of the inflatable cushion 30, or even to gradually deflate the inflatable cushion 30.

Optionally, a patient data record associated to the patient by a unique patient identification number enables previously obtained (and confidentially stored) signals representing a proxy pain level obtained during transfer of the unique patient to be used to determine whether a patient is experiencing pain during a new transfer. For example, a patient suffering from a broken arm will usually experience pain triggered using the same types of movements between the first and second patient support. Optionally, a video camera 32 maybe used to monitor patient transfer and compare the patient posture to postures of previous transfers that resulted in a change in the first signal from a first level to a second level indicating patient was experiencing pain. If the processing device 14 tracks the patient posture obtained by the video camera 32 during the transfer with a record of a previous transfer stored in the patient data record, the processing device 14 may determine that the patient is at risk of suffering from being put into a painful posture, and accordingly communicate to a patient transfer device to cause it to perform a first physical adaptation to prevent patient pain from occurring. Prior records in the patient data record of galvanic skin conductance, heart rate, breathing rate can be compared in a similar way.

According to an embodiment, the input unit 12 is therefore further configured to obtain a patient data record of the patient prior to the transfer of the patient, and the processing unit 14 is further configured to detect the change in the first signal between the first and the second levels based additionally on a portion of the data comprised in the patient data record.

Optionally, the output unit 16 is further configured to transmit the adaptation signal to perform a first physical adaptation of a patient transfer device wherein the adaptation signal causes a patient transfer unit to initiate an actuator configured to transfer the patient between the first patient support and the second patient support.

The patient transfer device can be located exclusively on the first patient support, exclusively on the second patient support, as a separate element placed in between, or across the first and second patient supports. The patient transfer device may take many different forms. For example, the patient transfer device is a semi-rigid surface with inflatable chambers. In other words, the support has a semi-rigid top layer that can be flexed to become either curved or flat. It is supported by several inflatable or deflect what chambers which are used to flex or flatten the semi-rigid top layer.

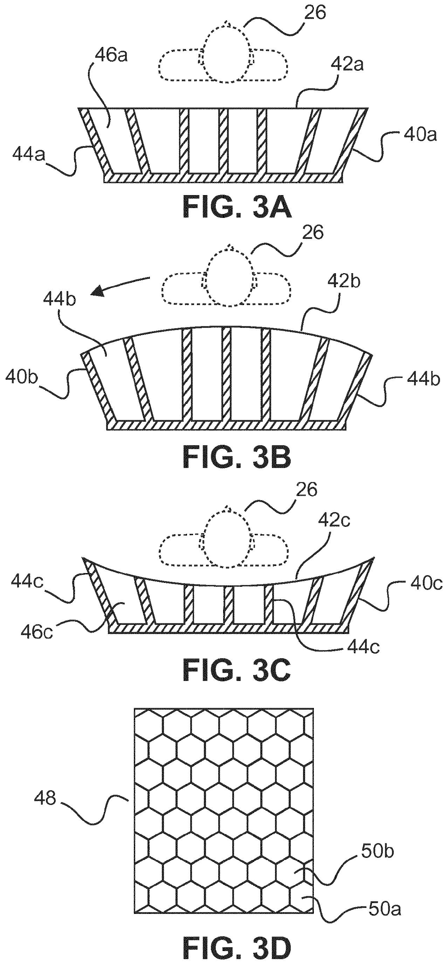

Figures 3a)-c) schematically illustrates side-views of an embodiment of a patient transfer device with a lamellar structure made from a resilient airtight rubber, or plastic, for example, in different configurations. In figure 3 a), the array of lamellae 44a form flexible or semi-rigid walls enclosing inflatable chambers 46a. The inflatable chambers may be progressively inflated to enable the top surface 42a of the patient transfer device to be substantially planar. This enables a patient 26 to be pulled off the patient support without rolling them. In figure 3b), the inflatable chambers 46b have been pressurised to cause the lamellae 46b to expand outwards and the semi-rigid central lamella to be stretched upwards by the airtight top surface 42b, which has assumed a convex characteristic in comparison with that of 42a. In other words, in figure 3b) the outermost inflatable chambers 46b have a lower pressure than the central inflatable chambers. This enables a patient 26 to be rolled off the patient support 40 more easily when it is in the state shown in figure 3b). In figure 3c), the most central inflatable chambers have a lower pressure than the outermost inflatable chambers 46c, causing the top surface 42c to assume a concave characteristic enabling a patient 26 to be more securely held in the patient support. Indeed, in this configuration the illustrated patient support would be ideal for use as a CT scanner or MRI scanner couch (second patient support).

Alternatively or in combination, the lamellae 44 of the patient transfer device illustrated in figures 3a)-c) are stretchable using an array of small actuators (such as solenoids, hydraulic pistons, gas pistons) which are used to flex and flatten the surface of the patient support 42.

Optionally, the patient transfer device comprises a plurality of lamellae that are supported by actuators that are configured to lift and rotate one or more of the lamellae individually. The mechanism is used to adapt all or a subset of the lamellae to form either a curved or flat service. The gaps between the lamellae may be closed with flexible material so that closed surface is provided, facilitating cleaning and avoiding the ingress of liquids.

Optionally, the patient transfer device comprises a laterally tiltable surface configured to provide a declining slope in-between the first and second patient support (when transferring a patient from the first to the second patient support). Optionally or in combination, the patient transfer device comprises a laterally tiltable surface configured to provide a declining slope in-between the second patient support and the first patient support (when transferring a patient from the second patient support to the first patient support). The laterally tiltable surface is, for example, attached using hinge at one side of the patient support and is actuated using, for example, a hydraulic or gas piston attached to the underside of the laterally tiltable surface, or a rack and pinion connection.

Optionally, the patient transfer device comprises a mattress having a plurality of inflatable and/or deflatable elements. For example, figure 3d) illustrates a plan view of a mattress 48 functioning as a patient transfer device for placing on top of a first patient support and/or a second patient support. The mattress comprises a plurality 50a, 50b of inflatable and/or deflatable actuators. The actuators may be circular, or tessellated square, triangular, rectangular, or hexagonal inflatable cushions, for example.

In use, such a patient transfer device may initially be in a latent state with all actuators either inflated or deflated, providing an approximately flat surface. Then, a subset of the inflatable actuators may be inflated on a side it is intended to roll the patient away from. Optionally, a first subset of inflatable actuators may be inflated to a first height that is greater than a second subset of inflatable actuators (in other words, the inflatable actuators can be used to provide an approximation that of the surface). Still further, such a patient transfer device may be configured so that a subset of the inflatable actuators are provided in an ad hoc pattern matched to the shape of a patient. For example, inflatable actuators underlying a patient's legs, middle body, arms, and/or head may form a third subset inflatable actuators that are inflated to a lower degree than surrounding inflatable actuators. When it is desired to move the patient, the actuators not comprised in the third subset on one side of the patient transfer device are deflated to match the height of the actuators of the third subset.

Optionally, the patient transfer device comprises a motorised roller platform having a plurality of motorised rollers arranged in the lateral plane. The patient may be placed onto one side of motorised roller and transferred across from the first patient support the second patient support. Alternatively, the patient transfer device may comprise a rubber conveyor belt between the first patient support and second patient support.

Optionally, the patient support is equipped with a flexible resilient sheet (comprised, for example, of silicone rubber) having a plurality of inflatable longitudinal cavities. These cavities are pressurised in sequence to form a surface wave. Optionally, the flexible resilient sheet is partitioned into two, three, four, or more sections along the length of the patient support to enable the surface wave to be created independently at different longitudinal locations along the bed. The surface wave, or subsets have surface waves thereby generated can move, or assist the movements of patient from the first patient support the second patient support and back again.

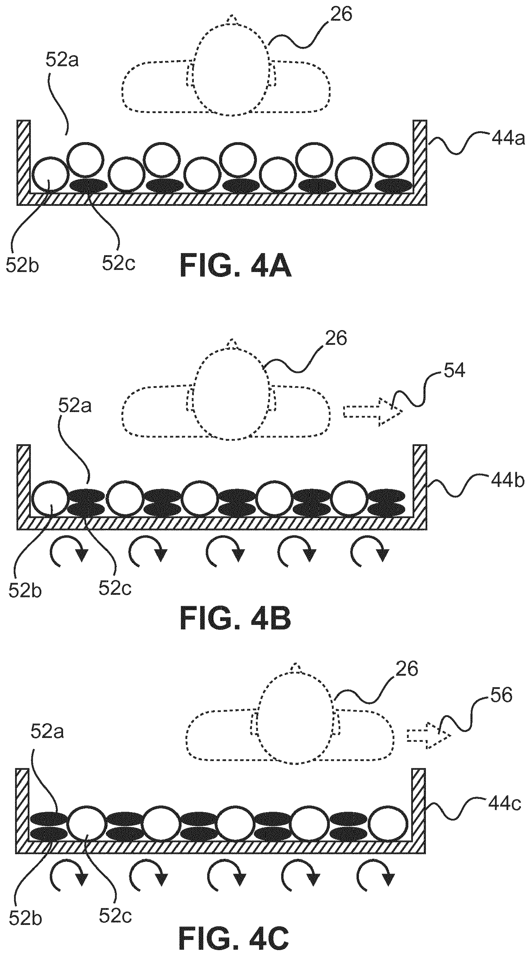

Figures 4a) to 4e) schematically illustrate a side view of an embodiment of a patient transfer device 44 according to an embodiment having sequentially inflatable longitudinal pockets. In Figure 4a), the sequentially inflatable longitudinal pockets are provided in triplets. One triplet of pockets is designated 52a, 52b, 52c. A patient 26 rests on the inflated longitudinal pockets.

Each triplet may be controlled (by the application or withdrawal of a pressurised fluid) to inflate sequentially to translate the patient.

Figure 4a) illustrates a starting condition in which the first 52a and second 52b set of longitudinal pockets are inflated, and a third set of longitudinal pockets 52c are deflated.

Figure 4b) illustrates a first intermediate condition in which the first set 52a of longitudinal pockets have additionally been deflated.

Figure 4c) illustrates a third intermediate condition in which the second set 52b of longitudinal pockets have been deflated, and the third set 52c of longitudinal pockets have been inflated.

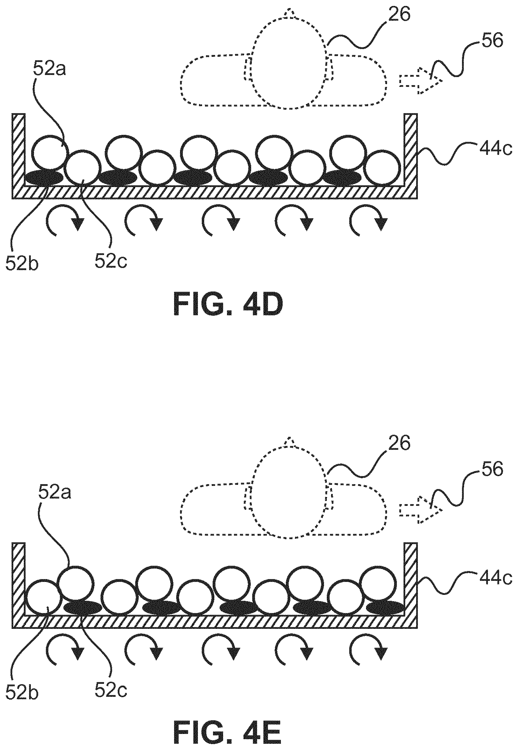

Figure 4d) illustrates a fourth intermediate condition in which the first set 52a of longitudinal pockets are inflated.

Figure 4e) illustrates a fifth intermediate condition in which the second set 52b of longitudinal pockets are inflated, and the third set 52c of longitudinal pockets are deflated.

In this way, additional inflation and deflation of the first set of longitudinal pockets in synchrony results in a clock-wise motion of the upper side of the tubes of the first set of longitudinal pockets, translating the patient to the right hand side of the arrangement illustrated in Figs 4a)-e). Optionally, the triplets are controlled to perform their collective "rotation" with a phase lag that increases from the left to the right hand side of the arrangement illustrated in Figures 4a) - e). This simulates a "surface wave" that moves the patient 26 further in the direction of arrow 56. By repetition of the above-outlined sequence, a patient can be transferred between a first and a second patient support. Of course, other sequences may be provided which provide a similar surface-wave effect, and also a greater or smaller number of inflatable actuators. The inflation and deflation of the first, second, third, sets of inflatable elements may be finely controlled to reduce patient pain during transfer. Such a patient transfer device may also be presented in an embodiment in which a first active sheet is fixed to the first patient support and the second active sheet is fixed to the second patient support. Both active sheets are operated in unison to transfer the patient with waves at the upper surfaces.

Optionally, any of the above transfer mechanisms may be complemented with movement sensors that measure the lateral position of the patient during automatic transfer and stop the patient transfer as soon as the patient has arrived to the desired position. The position sensors such as linear/rotary/and tilt sensors are used for autonomous correct position check on the scanner table. Position sensors can detect the movement of the person in straight-line using linear sensors pies and a movement using rotational sensors.

Patient transfer devices comprising inflatable chambers to move the patient may be used to provide a flat or a curved patient support or for pain relief (for example, in burn units, or for decubitus patients). For this purpose, the inflatable chambers maybe additionally structured long length the patient support, effectively providing a grid of chambers at the surface of the patient support. Pain may be relieved by lowering or increasing the fill pressure of individual chambers beneath body parts that are particularly susceptible to pain. Optionally, body parts that are particular susceptible to pain may be identified from a patient data record. Optionally, body parts that are susceptible to pain may be dynamically monitored using a pain sensor, and the pain reduced using a control loop.

Accordingly, the input unit 12 is further configured to receive a second signal representing a location of the first physical adaptation of the patient transfer device relative to the patient, and to receive a third signal representing a proxy for a pain level experienced by a patient after a second physical adaptation of the patient transfer device and a fourth signal representing a location of the second physical adaptation of the patient transfer device relative to the patient. The processing unit 14 is further configured to associate the second signal and the first signal, to perform the second physical adaptation of the patient transfer device, to associate the third signal and the fourth signal.

The output unit 16 is further configured to transmit a fifth signal to perform a third physical adaptation of the patient transfer device based on the change of the first and third signals.

Optionally, the output device 16 is configured to transmit the fifth signal to the patient transfer device to perform a physical adaptation of the patient transfer device comprising one or more of: transmitting a signal to (i) halt or reverse an inclination of a laterally tilting patient transfer device, to (ii) halt or reverse the adjustment of a plurality of lamellae within a patient transfer device, to (iii) decrease or increase the resilience of a subset of patient support actuator cells; or to halt or reverse a plurality of rollers within a patient transfer device.

According to a second aspect, there is provided system 60 for patient pain detection and reduction during a transfer between a first 62 and a second 64 patient support using a patient transfer device 66 comprising:
- a patient transfer device 66 configured to move a patient in-between a first 62 and a second 64 patient support;
- a one or more sensors 68 configured to obtain a first signal representing a proxy for a pain level experienced by a patient associated a patient transfer in-between the first and a second patient supports, and/or when the patient is stationary on the first or second patient supports; and
- a device 10, 70 according to the first aspect was optional embodiments configured to output an adaptation signal to the patient transfer device 66 to generate a physical adaptation of the patient transfer device, to change the rate of the patient transfer, or to halt the patient transfer device, in response to the change in the signal to a second level indicating that the patient has begun to experience pain.

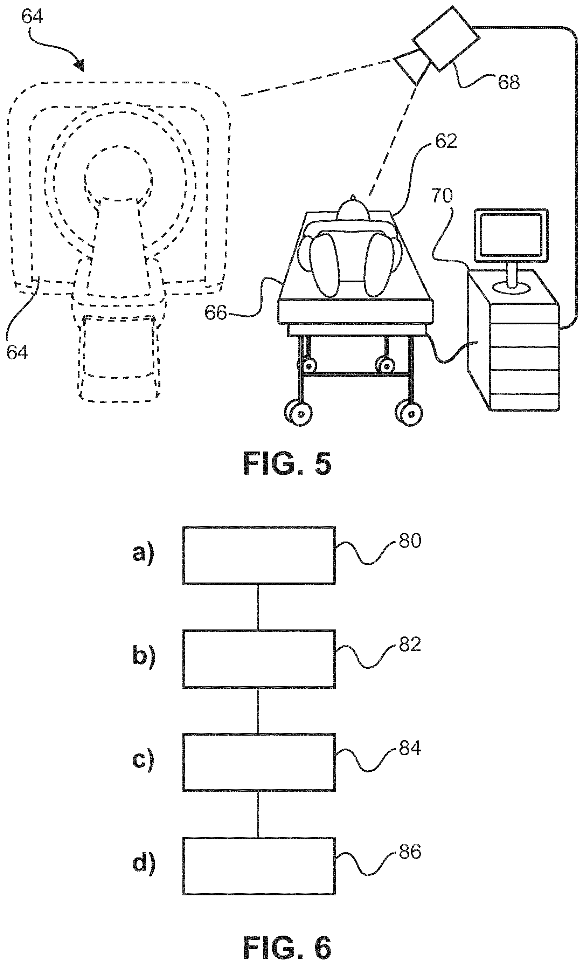

Figure 5 schematically illustrates a system according to the second aspect. The illustration, a first patient support is a wheeled patient carrier, and the second patient support is a MRI scanner support. The patient transfer device 66 illustration is incorporated into a mattress of the first patient support. The device 70 comprises a device according to the first aspect which receives video signals from the video camera 68. Therefore, in use, the first patient support 62 is brought substantially into contact, or is positioned close to, the second patient support. The patient transfer area is monitored using the device 70 by processing the signals received from the video camera 68. If the video camera receives an image of a patient changing shape or expression in such a way as to indicate the presence of an increase in pain, then the device 70 sends a control signal to the patient transfer device 66 to cause the patient transfer device 66 to perform the first physical adaptation, or to halt the patient transfer device in response to the change in signal to a second level indicating that the patient has begun to experience pain.

According to a third aspect, there is provided a method for patient pain detection and reduction before, during, or after a transfer between a first and a second patient support using a patient transfer device comprising:
a) obtaining 80, via one or more sensors, a first signal representing a proxy for a pain level experienced by a patient before, during, or after the transfer of the patient using a patient transfer device between the first and the second patient support ;
b) monitoring 82, via a processor, the first signal representing a proxy for pain level;
c) detecting 84, via the processor, a change in the first signal from a first level, to a second level indicating that the patient is experiencing a greater degree of pain
d) performing 86, a first physical adaptation of the patient transfer device, or halting the patient transfer device, in response to the change in the first signal to a second level indicating that the patient is experiencing a greater degree of pain.

Figure 6 schematically illustrates the method according to the third aspect. According to a fourth aspect, there is provided a computer program element for controlling a processing unit and/or system of the first and/or third aspect, which, when the computer program element is executed by the processor and/or system, is adapted to perform the method of the second aspect.

According to a fifth aspect, there is provided a computer readable medium having stored the computer program element of the fourth aspect.

A computer program element might therefore be stored on a computer unit, which might also be an embodiment of the present invention. This computing unit may be adapted to perform or induce performance of the steps of the method described above.

Moreover, it may be adapted to operate the components of the above-described apparatus.

The computing unit can be adapted to operate automatically and/or execute orders of a user. A computer program may be loaded into the working memory of a data processor. The data processor may thus be equipped to carry out the method of the invention.

This exemplary embodiment of the invention covers both the computer program that has the invention installed from the beginning, and a computer program that by means of an update turns an existing program into a program that uses the invention. A computer program may be stored and/or distributed on a suitable medium, such as an optical storage medium, or a solid state medium supplied together with, or as part of other hardware, but may also be distributed in other forms, such as via the Internet or other wired or wireless telecommunication systems.

However, the computer program may also be presented over a network like the World Wide Web, and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It should to be noted that embodiments of the invention are described with reference to different subject-matters. In particular, some embodiments are described with reference to method-type claims, whereas other embodiments are described with reference to device-type claims. However, a person skilled in the art will gather from the above, and the following description that, unless otherwise notified, in addition to any combination of features belonging to one type of subject-matter, other combination between features relating to different subject-matters is considered to be disclosed with this application.

All features can be combined to provide a synergetic effect that is more than the simple summation of the features.

While the invention has been illustrated and described in detail in the drawings and foregoing description, such illustration and description are to be considered illustrative or exemplary, and not restrictive. The invention is not limited to the disclosed embodiments.

Other variations to the disclosed embodiments can be understood, and effected by those skilled in the art in practicing the claimed invention, from a study of the drawings, the disclosure, and the dependent claims.

In the claims, the word "comprising" does not exclude other elements or steps, and the indefinite article "a" or "an" does not exclude a plurality. A single processor, or other unit, may fulfil the functions of several items recited in the claims. The mere fact that certain measures are recited in mutually different dependent claims does not indicate that a combination of these measures cannot be used to advantage. Any reference signs in the claims should not be construed as limiting the scope.

## Claims

1. A device (10) for enabling patient pain detection and reduction when using a patient transfer device configured to transfer a patient between a first and a second patient support comprising:
- an input unit (12);
- a processing unit (14); and
- an output unit (16);
wherein the input unit (12) is configured to obtain, via one or more sensors, a first signal representing a proxy for a pain level experienced by a patient before, during, or after the transfer of the patient using a patient transfer device between the first and the second patient support;
wherein the processing unit (14) is configured to monitor the first signal representing a proxy for pain level, to detect a change in the first signal from a first level, to a second level indicating that the patient is experiencing a greater degree of pain; and
wherein the output unit (16) is configured to transmit an adaptation signal to a patient transfer device to cause the patient transfer device to perform a first physical adaptation, to change the rate of the patient transfer, or to halt the patient transfer in response to the change in the first signal to a second level indicating that the patient is experiencing a greater degree of pain.

2. The device (10) according to claim 1,
wherein the first signal received by the input unit (10) comprises a vital sign of the patient comprised of: galvanic skin conductance, heart rate, or breathing rate.

3. The device (10) according to one of claims 1 or 2,
wherein the input unit (12) is further configured to receive a video signal from a video camera or a depth camera monitoring the patient transfer area; and
wherein the first signal is derived from at least a portion of the video signal and represent at least a posture of the patient, a facial expression of the patient, an eye movement, and/or a change in pupil size of the patient.

4. The device (10) according to one of the preceding claims,
wherein the processing unit (14) is further configured, when detecting, via the processor, a change in the first signal from the first level, to the second level, to compare the first signal to a first example signal representing an absence of pain, and to compare the first signal to a second example signal representing the presence of pain.

5. The device (10) according to one of the preceding claims,
wherein the input unit (12) is further configured to obtain a patient data record of the patient prior to the transfer of the patient, and the processing unit (14) is further configured to detect the change in the first signal between the first and the second levels based additionally on a portion of the data comprised in the patient data record.

6. The device (10) according to one of the preceding claims,
wherein the output unit (16) is further configured to transmit the adaptation signal to perform a first physical adaptation of a patient transfer device wherein the adaptation signal causes a patient transfer unit to initiate an actuator configured to transfer the patient between the first patient support and the second patient support.

7. The device (10) according to one of the preceding claims,
wherein the input unit (12) is further configured to receive a second signal representing a location of the first physical adaptation of the patient transfer device relative to the patient, and to receive a third signal representing a proxy for a pain level experienced by a patient after a second physical adaptation of the patient transfer device and a fourth signal representing a location of the second physical adaptation of the patient transfer device relative to the patient;
wherein the processing unit (14) is further configured to associate the second signal and the first signal, to perform the second physical adaptation of the patient transfer device, to associate the third signal and the fourth signal; and
wherein the output unit (16) is further configured to transmit a fifth signal to perform a third physical adaptation of the patient transfer device based on the change of the first and third signals.

8. The device (10) according to one of the preceding claims,
wherein the output unit (16) is configured to transmit the fifth signal to the patient transfer device to perform a physical adaptation of the patient transfer device comprising one or more of: transmitting a signal to (i) halt or reverse an inclination of a laterally tilting patient transfer device, to (ii) halt or reverse the adjustment of a plurality of lamellae within a patient transfer device, to (iii) decrease or increase the resilience of a subset of patient support actuator cells; or to halt or reverse a plurality of rollers within a patient transfer device.

9. A system (60) for patient pain detection and reduction during a transfer between a first and a second patient support using a patient transfer device comprising:
- a patient transfer device (66) configured to move a patient in-between a first and a second patient support;
- a one or more sensors (68) configured to obtain a first signal representing a proxy for a pain level experienced by a patient associated a patient transfer in-between the first and a second patient supports, and/or when the patient is stationary on the first or second patient supports; and
- a device (70) as defined in one of claims 1 to 8 configured to output an adaptation signal to the patient transfer device (68) to generate a physical adaptation of the patient transfer device, to change the rate of the patient transfer, or to halt the patient transfer device, in response to the change in the signal to a second level indicating that the patient has begun to experience pain.

10. The system (60) according to claim 9,
wherein the one or more sensors (68) comprise one or more of a galvanic skin conductance monitor, a heart rate monitor, a breathing rate monitor.

11. The system (60) according to one of claims 9 or 10,
wherein the one or more sensors (68) comprise a video camera and/or a depth camera, wherein the first signal is derived from a portion of a video signal representing at least a posture of the patient, a facial expression of the patient, or an eye movement or change in pupil size of the patient.

12. The system (60) according to one of claims 9 to 11,
wherein the patient transfer device (66) comprises a resilient member having a plurality of longitudinal gas-tight pockets capable of sequenced inflation to generate a surface wave for transferring the patient between the first and the second patient support, wherein performing a physical adaptation of the patient transfer device further comprises:
inflating a first subset of the longitudinal gas-tight pockets of the plurality of longitudinal gas-tight pockets; and
if the processor does not detect a change in the signal from the first level to a second level, deflating the first subset of the longitudinal gas-tight pockets of the plurality of longitudinal gas-tight pockets and inflating a second subset of the longitudinal gas-tight pockets of the plurality of longitudinal gas-tight pockets to transfer the patient a unit of distance between the first and/or second patient supports.

13. A method for patient pain detection and reduction before, during, or after a transfer between a first and a second patient support using a patient transfer device comprising:
a) obtaining (80), via one or more sensors, a first signal representing a proxy for a pain level experienced by a patient before, during, or after the transfer of the patient using a patient transfer device between the first and the second patient support ;
b) monitoring (82), via a processor, the first signal representing a proxy for pain level;
c) detecting (84), via the processor, a change in the first signal from a first level, to a second level indicating that the patient is experiencing a greater degree of pain
d) performing (86), via an output device, a first physical adaptation of the patient transfer device, changing the rate of the patient transfer, or halting the patient transfer device, in response to the change in the first signal to a second level indicating that the patient is experiencing a greater degree of pain.

14. A computer program element comprising instructions which, when executed by a computer processor, causes the computer processor to perform the steps of claim 13.

15. A computer readable medium comprising the computer program element as defined in claim 14.
